# DEMANDE DE BREVET EUROPEEN

(11) **EP 1 582 197 A1**
(43) Date de publication de la demande: **05.10.2005**
(21) Numéro de dépôt: 05290205.3
(22) Date de dépôt: 31.01.2005
(51) Int. Cl.: A61K 7/043

(54) **Composition cosmétique pour ongles à résistance améliorée**

(30) Priorité: 01.04.2004 FR 0403449
(71) Demandeur: Fiabila, 28130 Maintenon (FR)
(72) Inventeur: Malnou, Alain, 27350 Routot (FR); Martinez, Francisco, 28000 Chartres (FR)
(74) Mandataire: Laget, Jean-Loup

(57) **Abrégé**

L'invention concerne une composition cosmétique pour ongles comprenant de 0,1 % à 20 % en poids de particules d'oxydes non agglomérées ayant une taille moyenne inférieure à 100 nm, lesdites particules ne présentant pas de groupes fonctionnels réactifs à leurs surfaces et n'ayant subi aucun traitement de surface.

L'utilisation de ces particules augmente la résistance à l'usure et à l'écaillage sans nuire au brillant et à la transparence du film, ni augmenter notablement sa viscosité.

## Description

L'invention concerne une composition pour ongles comprenant des particules nanométriques de caractéristiques particulières, et son utilisation comme vernis, coloré ou non.

Typiquement, une composition de vernis à ongles comporte un polymère filmogène principal, généralement cellulosique et plus précisément nitrocellulosique, des polymères secondaires comme les résines polyesters, acryliques ou de condensation avec le tosylamide, un ou plusieurs plastifiants et des solvants. Ce mélange de base peut également contenir des matières colorantes, des agents de suspension et des additifs tels que des agents anti-UV, des agents mouillants, des agents de glissance, des agents hydratants, des parfums, etc...

Toutes ces formulations cherchent à optimiser les performances telles qu'une application bien arrondie, un temps de séchage court et un très bon brillant. Le formulateur essaie ensuite de garder cet aspect en conférant à la formulation également une bonne résistance à la rayure, à l'usure, à l'écaillage, à l'eau et aux détergents. Ceci doit se traduire par une bonne adhérence et un bon compromis dureté / souplesse dudit vernis.

De nombreuses solutions ont été proposées pour répondre à ces diverses contraintes.

L'ajout de charges minérales améliore ce compromis mais au détriment de la brillance et de la transparence.

Une autre solution est de formuler un film avec une dureté relativement basse pour avoir une bonne adhérence sans écaillage, l'usure du film étant alors minimisée par l'utilisation de cire et d'additifs siliconés. Cette solution n'évite cependant pas une perte de brillant à l'origine et durant la vie du film.

D'autres formulations utilisent des silices colloïdales ayant ou non subi un traitement de surface par des silanes. Ces constituants présentent l'inconvénient que les interactions entre les particules de silice nanométriques sont très fortes du fait de la présence des fonctions de surface, notamment de type OH, et il est difficile d'éviter les agrégats pendant la fabrication du vernis. Il est ainsi quasiment impossible de parvenir à une dispersion homogène, réellement nanométrique mono disperse, n'ayant pas d'incidence sur la viscosité, sur la transparence et sur le brillant du film. En particulier, une augmentation significative de la viscosité est fréquemment observée (voir exemple comparatif 5 présenté plus loin), ce qui pose notamment des problèmes d'application du vernis, ainsi que de brillant et de stabilité du film.

Par conséquent, un but de la présente invention est de proposer une composition cosmétique pour les ongles, présentant une résistance physique renforcée, sans nuire au brillant et à la transparence du film, ni à sa viscosité lors de l'application.

Il a été découvert que l'utilisation de particules nanométriques, en particulier d'oxydes, ne présentant pas de fonctions de surface, notamment de fonctions -OH, -CO ou -COOH détectables par exemple par mesure infra rouge, confère, même à de faibles concentrations, des propriétés de résistance intéressantes à des compositions cosmétiques pour les ongles, sans augmenter la viscosité de la composition de base.

A cet effet, la composition cosmétique pour ongles selon la présente invention comprend de 0,1 % à 20 % en poids de particules non agglomérées ayant une taille moyenne inférieure à 100 nm, lesdites particules ne présentant pas de groupes fonctionnels réactifs à leurs surfaces et n'ayant subi aucun traitement de surface. L'ajout de ces "nanoparticules" confère avantageusement à la composition une viscosité ne dépassant pas 1,5 fois la viscosité initiale (c'est-à-dire en l'absence desdites particules).

L'affinité de ces particules pour les polymères de la composition cosmétique pour les ongles est supérieure à l'affinité des particules entre elles; ceci permet ainsi la dispersion des particules dans le milieu sans agglomération. La dispersion homogène dans le vernis conduit également à une dispersion homogène dans la couche de vernis sec sur l'ongle. Ceci induit les propriétés recherchées pour la composition de l'invention, à savoir : un meilleur compromis dureté / souplesse et une dureté de surface améliorée sans perte de brillant.

De préférence ces particules sont des particules d'oxydes choisis parmi les oxydes de zinc, d'aluminium, de titane, de cérium, de silicium, de manganèse, de bismuth, de cuivre, de zirconium ou de fer, ou un mélange homogène de plusieurs de ces oxydes, tels que des oxydes mixtes. On peut aussi utiliser des particules de carbures comme les carbures de silicium, de nitrures comme les nitrures de silicium, d'aluminium ou de bore.

Ces particules sont fabriquées de telle façon qu'elles ne présentent pas de groupes fonctionnels à leurs surfaces, comme par exemple des groupes - OH. Cette fabrication particulière est décrite en détail dans le brevet US 5 749 937. Cette propriété importante permet de disperser lesdites particules dans un mélange de polymères en solution dans des solvants organiques, tels que ceux utilisés dans les vernis à ongles par exemple, en évitant les agglomérations entre particules.

Dans le cas de mélange d'oxydes, ces mélanges sont obtenus de façon très homogène pendant la fabrication selon le procédé précité.

De manière avantageuse, les particules sont des oxydes incolores d'aluminium, de zinc, de zirconium et/ou de silicium, de préférence des particules homogènes d'oxydes mixtes comprenant de 1 à 99 % en poids d'oxyde d'aluminium, de zirconium ou de titane et de 1 à 99 % en poids d'oxyde de silicium.

De manière encore préférée, les particules utilisées dans les compositions selon l'invention sont des particules homogènes d'oxydes mixtes comprenant entre 10 et 70 % en poids d'oxyde d'aluminium et entre 30 et 90 % en poids d'oxyde de silicium.

La dureté de ces particules exprimée en Moh est avantageusement supérieure à 5 et de préférence supérieure à 6.

Ces particules présentent avantageusement un indice de réfraction voisin de celui du mélange de polymères de la composition cosmétique pour ongles pour obtenir le maximum de transparence. Typiquement pour des particules constituées d'oxydes d'aluminium et de silicium, cet indice se situe entre 1,4 et 1,8, mais peut être compris entre 1,4 et 1,7, et de préférence entre 1,5 et 1,6 suivant la taille des particules. Ces particules sont de préférence incolores pour une gamme d'utilisation plus large de la composition cosmétique pour les ongles, les particules colorées étant réservées à des effets spécifiques dans des teintes particulières.

Les particules utilisées sont de préférence de forme sphérique et présentent une taille moyenne inférieure à 50 nm et de manière encore préférée inférieure à 20 nm. Ces tailles sont calculées à partir des surfaces obtenues par microscopie électronique. La répartition granulométrique de ces nanoparticules peut être mono modale ou multi modale, mais une répartition granulométrique mono modale et resserrée est préférée.

Les compositions cosmétiques pour ongles selon la présente invention comprennent avantageusement de 0,1 à 5 % en poids, de préférence entre 0,5 et 5 % en poids des particules décrites ci-dessus.

Elles trouvent une utilisation intéressante en tant que vernis à ongles coloré, appliqué en une ou plusieurs couches, en tant que primaire d'adhérence sur l'ongle ou en tant que vernis à ongles de finition coloré ou non.

Une composition pour ongles préférée de l'invention comprend :
- un polymère filmogène principal, de préférence la nitrocellulose,
- un polymère filmogène secondaire, de préférence une résine polyester ou une résine acrylique,
- un ou plusieurs plastifiants,
- un ou plusieurs solvants,
- 0,5 à 5 % de particules ayant une taille inférieure à 80 nm ne présentant pas de groupes fonctionnels réactifs à leurs surfaces et n'ayant pas subi de traitement de surface.

La présente invention est illustrée par les exemples non limitatifs ci-après, regroupés dans le tableau 1.

### Exemples :

Différentes compositions de vernis à ongles ont été préparées, en incorporant dans les constituants de base différentes quantités et/ou type de nanoparticules :
L'exemple n° 0 est un essai blanc, c'est à dire une composition de vernis à ongles sans nanoparticules.
Les exemples n° 1 et n° 2 sont des essais avec deux types de nanoparticules, formées d'oxydes mixtes d'aluminium et de silicium de chez NanoProducts, Longmont, CO, USA, présentant respectivement des rapports atomiques Aluminium/Silicium de 6/95 (1) et de 44/56 (2), et utilisées à 2 % en poids dans la composition de vernis (représentant environ 1,2 % en volume de nanoparticules par rapport au volume des polymères de la composition).
Les nanoparticules (1) présentent une surface spécifique supérieure à 100 m²/g (méthode BET), un diamètre sphérique équivalent inférieur à 30 nm, et un indice de réfraction de 1,705.
Les nanoparticules (2) présentent une surface spécifique supérieure à 30 m²/g (méthode BET), un diamètre sphérique équivalent inférieur à 80 nm et un indice de réfraction de 1,606.
Ces nanoparticules (1) et (2) présentent une dureté (Moh) comprise entre 7 et 9.
L'exemple n° 3 est un essai réalisé en doublant la quantité de nanoparticules (1).
L'exemple n° 4 illustre un essai avec un film plus plastifié avec la même quantité de nanoparticules (1) que dans l'exemple n° 1.
Et l'exemple n° 5 est un exemple comparatif présentant l'utilisation de silice colloïdale (de taille voisine de 12 nm) comme connu de l'art antérieur.

**Tableau 1**

| **Exemple n°** | **0** | **1** | **2** | **3** | **4** | **5** (comparatif) |
|---|---|---|---|---|---|---|
| **Constituants** | | | | | | |
| Nitrocellulose (à 70 % dans alcool isopropylique) | 17 | 16,6 | 16,6 | 16,3 | 15,7 | 16,6 |
| Polyester (à 70 % dans acétate de butyle) | 8 | 7,8 | 7,8 | 7,7 | 8,3 | 7,8 |
| Acétyl citrate de tributyle | 6 | 5,9 | 5,9 | 5,8 | 6,2 | 5,9 |
| Acétate d'éthyle | 28 | 27,4 | 27,4 | 26,9 | 27,4 | 27,4 |
| Acétate de butyle | 38 | 37,3 | 37,3 | 36,3 | 37,3 | 38,5 |
| Stéaralkonium hectorite (100%) | 1 | 1 | 1 | 1 | 1 | 1 |
| Red 34 Ca lake (100%) | 2 | 2 | 2 | 2 | 2 | 2 |
| Nanoparticules (1) (à 40 % dans acétate de butyle) | - | 2 | - | 4 | 2 | - |
| Nanoparticules (2) (à 40 % dans acétate de butyle) | - | - | 2 | - | - | - |
| Silice colloïdale | - | - | - | - | - | 0,8 |

| **Résultats des tests** | | | | | | |
|---|---|---|---|---|---|---|
| Viscosité (a) | 845 | 921 | 954 | 982 | 880 | 1842 |
| Coeff. d'augmentation de viscosité | | (1,11) | (1,13) | (1,16) | (1,04) | (2,17) |
| Dureté (b) | 202 | 223 | 226 | 234 | 205 | 231 |
| Brillant initial (c) | 82 | 79 | 80 | 78 | 85 | 75 |
| Brillant final (d) | 60 | 75 | 78 | 81 | 70 | 65 |
| Souplesse (e) | 3 | 4 | 4 | 4 | 5 | 4 |
| (Les pourcentages sont tous exprimés en % en poids) | | | | | | |

Les tests indiqués dans le tableau 1 ci-dessus ont été réalisés dans les conditions suivantes :
(a) Viscosité en centipoises prise avec un appareil Brookfield mobile N° 3 vitesse 60 tours à 20°C.
   Le coefficient d'augmentation de viscosité est le rapport entre la valeur de viscosité de l'exemple et la valeur de viscosité du témoin (845 cP) sans nanoparticules.
(b) Dureté Persoz en secondes sur un film de 100 micromètres humide séché 24 heures.
(c) Sur un film dans les mêmes conditions, brillant sous un angle de 60°.
(d) Ce même film est soumis à une abrasion avec une brosse (6000 allers et retours). On mesure alors le % de brillant résiduel.
(e) Aspect du film après torsion sur un mandrin conique (5 : bon, 0 : craquelures importantes).

On peut remarquer que les nanoparticules utilisées n'augmentent pas de façon significative la viscosité (seulement quelques pour cent), il n'y a pas d'agrégation. En comparaison, l'ajout d'une quantité identique en poids de silice colloïdale (voir exemple comparatif 5) provoque l'augmentation considérable de la viscosité (celle-ci est plus que doublée), de la composition cosmétique.

Les nanoparticules augmentent la dureté du film par effet de charge. Par contre, il n'y a pas de perte de souplesse, le film semble plus résistant à l'élongation.

Les nanoparticules utilisées se comportent comme des charges inertes.

Ces petites charges donnent un brillant initial légèrement plus faible, que l'on peut aisément corriger par l'augmentation du plastifiant (exemple 4).

Les films chargés avec les particules selon l'invention présentent une bonne résistance à l'abrasion : le vernis à ongles aura donc une meilleure résistance à l'usure et gardera son brillant plus longtemps.

Il est également possible d'augmenter la plastification pour avoir un film plus adhérent et moins sensible à l'écaillage, tout en restant résistant à l'usure et à la perte de brillant.

## Revendications

1. Composition cosmétique pour ongles comprenant de 0,1 % à 20 % en poids de particules non agglomérées ayant une taille moyenne inférieure à 100 nm, lesdites particules ne présentant pas de groupes fonctionnels réactifs à leurs surfaces et n'ayant subi aucun traitement de surface.

2. Composition selon la revendication 1, **caractérisée en ce que** sa viscosité ne dépasse pas 1,5 fois la viscosité initiale.

3. Composition selon l'une quelconque des revendications précédentes, **caractérisée en ce qu'**elle comprend de 0,1 à 5 % en poids, de préférence entre 0,5 et 5 % en poids desdites particules.

4. Composition selon l'une quelconque des revendications précédentes, comprenant :
- un polymère filmogène principal, de préférence la nitrocellulose,
- un polymère filmogène secondaire, de préférence une résine polyester ou une résine acrylique,
- un ou plusieurs plastifiants,
- un ou plusieurs solvants,
- 0,5 à 5 % de particules ayant une taille moyenne inférieure à 80 nm ne présentant pas de groupes fonctionnels réactifs à leurs surfaces et n'ayant pas subi de traitement de surface.

5. Composition selon l'une quelconque des revendications précédentes, **caractérisée en ce que** les particules sont des particules d'oxydes choisis parmi les oxydes de zinc, d'aluminium, de titane, de cérium, de silicium, de manganèse, de bismuth, de cuivre, de zirconium ou de fer, ou un mélange homogène de plusieurs de ces oxydes.

6. Composition selon l'une quelconque des revendications précédentes, **caractérisée en ce que** la taille moyenne des particules est inférieure à 50 nm, de préférence inférieure à 20 nm.

7. Composition selon l'une quelconque des revendications précédentes, **caractérisée en ce que** les particules sont des particules homogènes d'oxydes mixtes comprenant de 1 à 99 % en poids d'oxyde d'aluminium, de zirconium ou de titane et de 1 à 99 % en poids d'oxyde de silicium.

8. Composition l'une quelconque des revendications précédentes, **caractérisée en ce que** les particules sont des particules homogènes d'oxydes mixtes comprenant entre 10 et 70% en poids d'oxyde d'aluminium et entre 30 et 90 % en poids d'oxyde de silicium.

9. Composition selon la revendication 8, **caractérisée en ce que** les particules ont un indice de réfraction compris entre 1,4 et 1,7, de préférence entre 1,5 et 1,6.

10. Utilisation de la composition selon l'une quelconque des revendications précédentes en tant que vernis à ongles coloré, appliqué en une ou plusieurs couches, en tant que primaire d'adhérence sur l'ongle ou en tant que vernis à ongles de finition coloré ou non.
